# EUROPEAN PATENT APPLICATION

(11) **EP 4 477 648 A1**
(43) Date of publication of application: **18.12.2024**
(21) Application number: 22925909.8
(22) Date of filing: 10.02.2022
(51) Int. Cl.: C07D 217/24, C07C 63/72, C07F 7/12, C07B 61/00

(54) **FLUOROISOQUINOLINE COMPOUND AND PRODUCTION METHOD THEREOF**

(71) Applicant: Carna Biosciences, Inc., Kobe-shi, Hyogo 650-0047 (JP)
(72) Inventor: KAWAHATA, Wataru, Kobe-shi, Hyogo 650-0047 (JP); GARNETT, Ian, Kobe-shi, Hyogo 650-0047 (JP); SHIMIZU, Tomohiro, Kobe-shi, Hyogo 650-0047 (JP); IRIE, Takayuki, Kobe-shi, Hyogo 650-0047 (JP)
(74) Representative: Kador & Partner Part mbB
(86) International application number: PCT/JP2022/005409
(87) International publication number: WO 2023/152888

(57) **Abstract**

The present invention provides a compound represented by formula (I), a production method thereof, a production intermediate of the same and a method for producing compound B using the compound (I). Compound B is useful as an intermediate for producing compound A that is a BTK inhibitor.

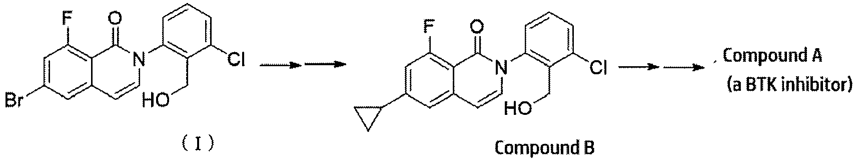

## Description

### Technical Field

The present invention relates to a method for producing a fluoroisoquinoline compound which is a production intermediate for producing a triazine derivative having a BTK inhibitory effect, and a production intermediate in the production thereof.

### Background Art

It is believed that compounds with Bruton's tyrosine kinase (BTK) inhibitory activity may be useful in the treatment of diseases in which BTK signaling is involved, such as cancer, B-cell lymphoma and chronic lymphocytic leukemia, and in the treatment of solid tumors in which p65BTK is expressed. It may also be useful in the treatment of allergic, autoimmune, and inflammatory diseases.

It has been found that triazine derivatives are particularly useful as BTK inhibitors, and Patent Literature 1 discloses the use of 2-(3-{4-amino-6-[(1-methyl-1H-pyrazol-4-yl)amino]-1,3,5-triazin-2-yl}-2-(hydroxymethyl)phenyl)-6-cyclopropyl-8-fluoroisoquinoline-1(2H)-one (compound A) as an example. And the Literature discloses a method for producing the compound A using a compound B which is an intermediate.

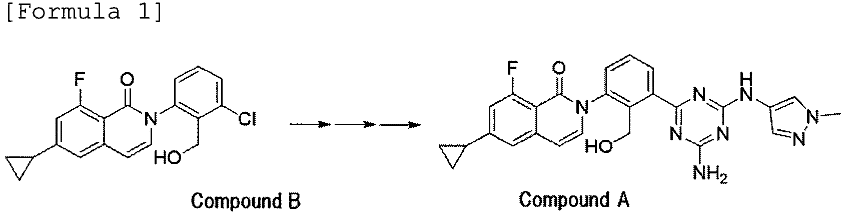

### Citation List

### Patent Literature

[Patent Literature 1]
WO 2015/012149

### Summary of Invention

### Technical Problem

To produce high quality compounds as pharmaceuticals on an industrial scale, it is necessary to solve problems such as reducing column chromatography purification processes, improving the operability and yield of production processes, increasing purity, reducing harmful solvents, in addition to developing new production intermediates that are compatible with actual production levels.

However, the production method of the compound B described in Patent Literature 1 is difficult to implement on a semi-industrial or industrial scale. Therefore, it was necessary to find a production method suitable for actual production by further improving the operability, purification efficiency, yield, etc. of the production.

The present invention provides a new production method of the compound B and a production intermediate useful in the production method. According to the present invention, the production processes are shortened and the compound B is obtained in high yield compared with the known synthesis method described in Patent Literature 1.

Furthermore, the production of the compound A using the compound B obtained by the present invention makes it possible to obtain a BTK inhibitor with a high overall yield and leads to a reduction in the production cost of the compound A.

### Solution to Problem

An object of the present invention is achieved by any one of the following inventions (1) to (9).
(1) A method for producing a compound B by reacting a compound (I) with cyclopropylboronic acid in the presence of a palladium catalyst and a base. 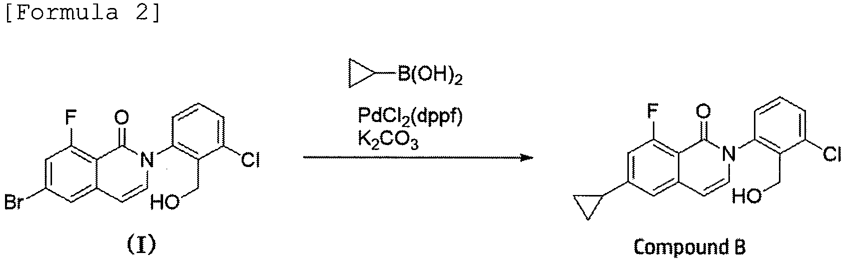
(2) The method for producing the compound B in the production method of the above (1), wherein according to the following scheme, a compound of the formula (II) is reacted with GDI, borane, and concentrated hydrochloric acid in order to produce the compound (I), and then the production method (1) is performed: 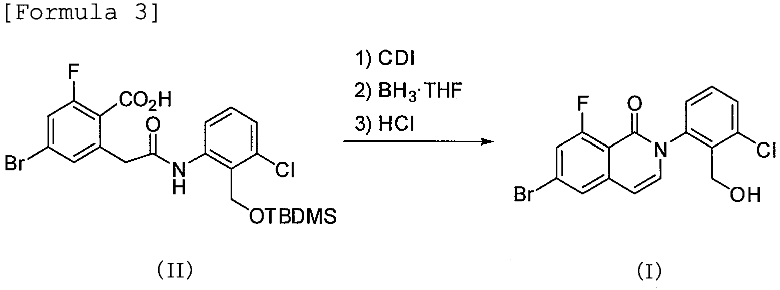 wherein TBDMS stands for tert-butyldimethylsilyl.
(3) The method for producing the compound B in the production method of the above (2), wherein according to the following scheme, a compound (III) is reacted with acetic anhydride, followed by reaction with a compound (VI) to produce the compound (II), and then the production method (2) is performed: 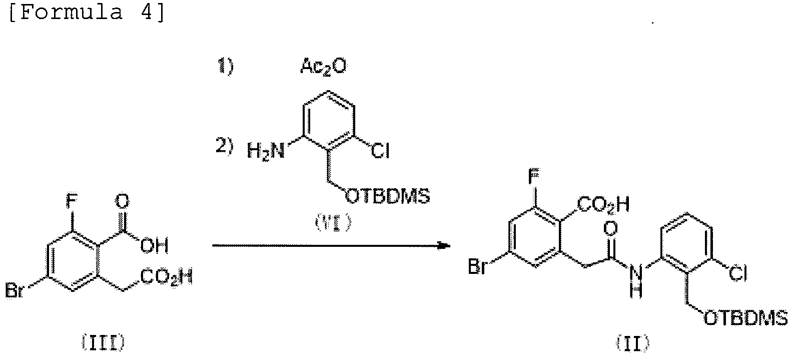 wherein TBDMS is defined as above.
(4) The method for producing the compound B in the production method of the above (3), wherein according to the following scheme, a compound of the formula (IV) is reacted with diethyl malonate (V) and further with concentrated sulfuric acid to produce the compound (III), and then the production method (3) is performed. 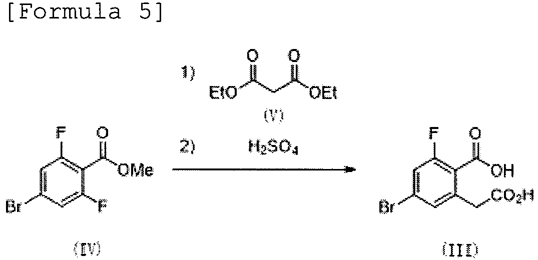
(5) A compound of the following formula (I), which is a production intermediate. 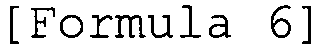 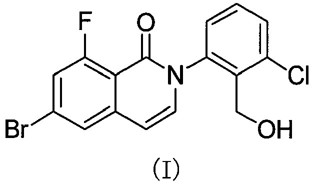
(6) A method for producing a compound of the formula (I), wherein according to the following scheme, a compound of the formula (II) is reacted with GDI, borane and concentrated hydrochloric acid in order: 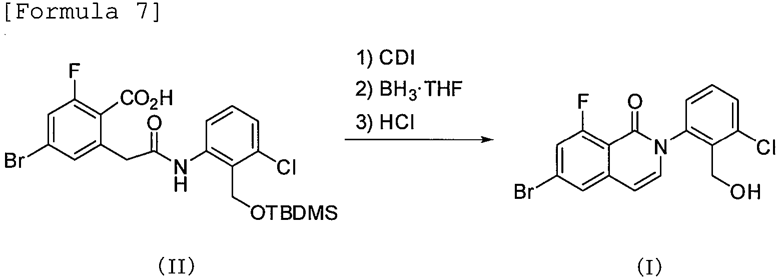 wherein TBDMS is defined as above.
(7) The method for producing the compound (I) in the production method of the above (6), wherein according to the following scheme, a compound (III) is reacted with acetic anhydride, followed by reaction with a compound (VI) to produce the compound (II), and then the production method (6) is performed: 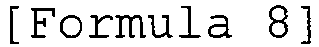 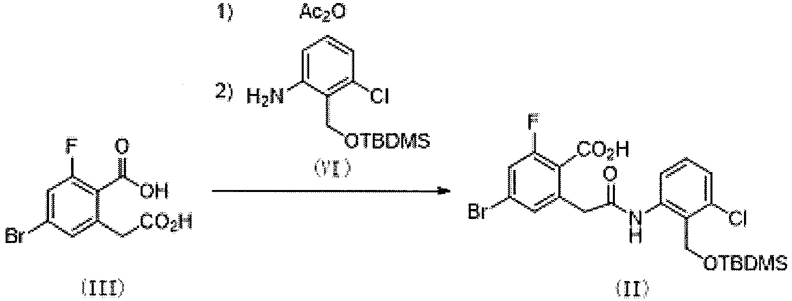 wherein TBDMS is defined as above.
(8) The method for producing the compound (I) in the production method of the above (7), wherein according to the following scheme, a compound (IV) is reacted with diethyl malonate (V), followed by treatment with concentrated sulfuric acid to produce the compound (III), and then the production method (7) is performed: 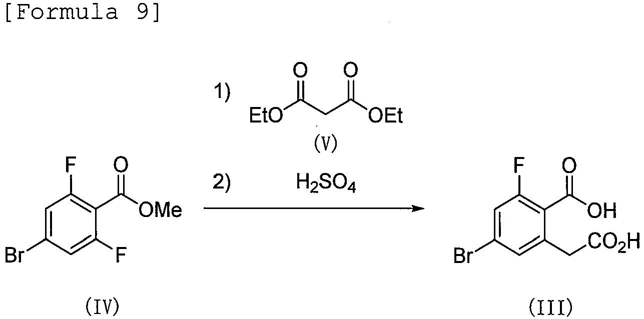
(9) A compound of the following formula (II) or (III) and a salt thereof, which are production intermediates: 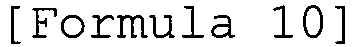 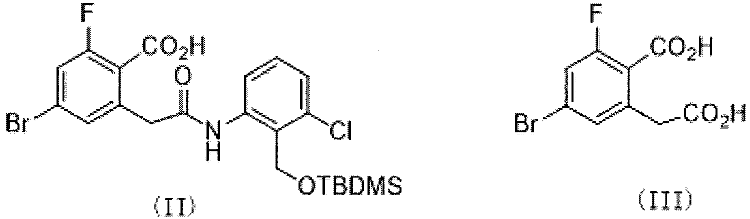 wherein TBDMS is defined as above.

### Advantageous Effect of Invention

The present invention provides a new synthesis method of a compound B and a synthesis intermediate useful in the synthesis method. According to the present invention, the production processes are shortened and the compound B is obtained in high yield compared with the known synthesis method described in Patent Literature 1.

Furthermore, the production of the compound A using the compound B obtained by the present invention makes it possible to obtain a BTK inhibitor with a high overall yield and leads to a reduction in costs.

### Description of Embodiments

The embodiment of the present invention is as follows.

Abbreviations and symbols used in the following description have the following meanings
CDI: 1,1'-Carbonyldiimidazole
DCM: Dichloromethane
THF: Tetrahydrofuran
DMF: Dimethylformamide
DMSO: Dimethyl sulfoxide
MeTHF: 2-Methyltetrahydrofuran
Pd-166: Bis(cyclohexyl)chlorophosphine palladium(II) dichloride
Pd(PPh₃)₄: Tetrakis (triphenylphosphine)palladium (0) PdCl₂ (dppf): [1,1'-Bis(diphenylphosphino)ferrocene]palladium(II) dichloride
TBDMS: Tert-Butyldimethylsilyl
TBME: Tert-Butyl Methyl Ether

(1) In the presence of a palladium catalyst and a base, a compound (I) is reacted with cyclopropylboronic acid to produce a compound B.

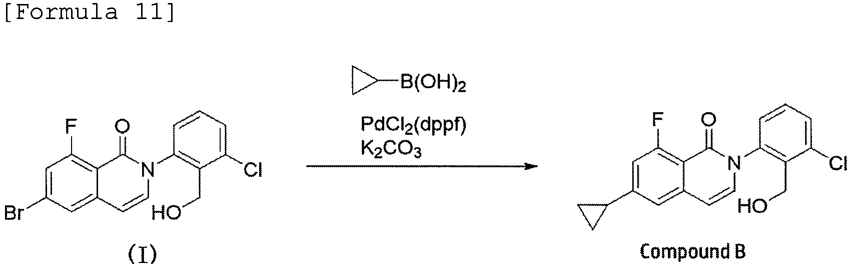

The solvent used in this reaction is selected from a mixture of toluene and water or ethyl acetate and water, the palladium catalyst used is PdCl₂(dppf) and can be either solvent-free or dichloromethane hydrate, and the added base is preferably potassium carbonate. A reaction temperature is preferably 70 to 100°C, more preferably 85 to 95°C. After the reaction, the compound B can be easily isolated as a solid and used in the next reaction, i.e. the reaction to produce a compound A, without any additional purifying operation.

(2) A method for producing the compound B, wherein a compound of the formula (II) is reacted with CDI, borane, and concentrated hydrochloric acid in order to produce the compound (I), and then the production method of the above (1) is followed.

The production of the compound (I) is performed, in more detail, through the following steps from the compound (II).

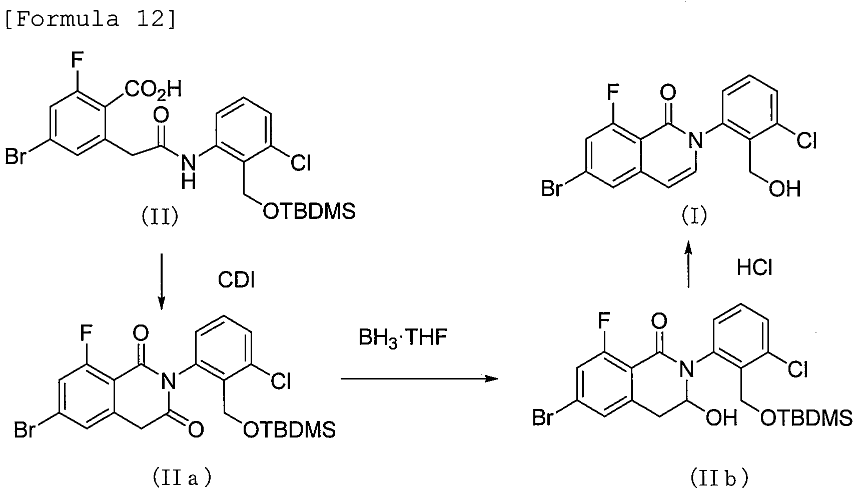

In the reaction to produce the formula (IIa), the solvent used is selected from the group consisting of dichloromethane (DCM), dimethylformamide (DMF), toluene, tetrahydrofuran (THF), and 2-methyltetrahydrofuran (MeTHF), and 1,1'-carbonyldiimidazole (CDI) may be used as a condensing agent. The resulting (IIa) may be isolated or used as a mixture for the next reaction.

In the reaction to produce the formula (IIb), the solvent used is selected from the group consisting of THF and MeTHF, sodium borohydride or borane may be used as the reducing agent, and a THF complex and a dimethyl sulfide (DMS) complex may be used as the borane. The resulting (IIb) may be isolated or used as a mixture for the next reaction.

In the reaction to produce the formula (I), the solvent used is selected from the group consisting of DCM and THF, and hydrochloric acid or trifluoroacetic acid may be used as the acid, and when the hydrochloric acid is used, concentrated hydrochloric acid is preferable. After producing the compound (I), the compound B can be produced according to the description of the above (1).

(3) A compound (III) is reacted with acetic anhydride, followed by reaction with a compound (VI) to produce the compound (II), and then the compound B is produced according to the production method of the above (2).

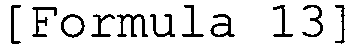

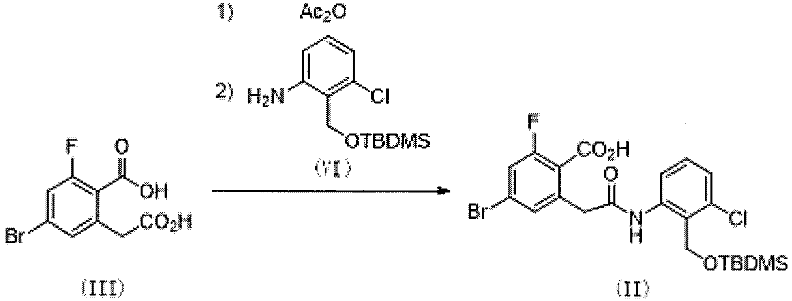

After reacting 1.05 to 1.5 equivalents of acetic anhydride with the compound (III) in a solvent such as toluene, 1.0 to 1.2 equivalents of aniline compound (VI) are reacted at room temperature for 0.5 to 1 hour to produce the target compound (II). After producing the compound (II), the compound B can be produced according to the description of the above (2).

(4) A compound of the formula (IV) is reacted with diethyl malonate (V), the resulting product is treated with concentrated sulfuric acid to produce the compound (III), and then the compound B is produced according to the production method of the above (3).

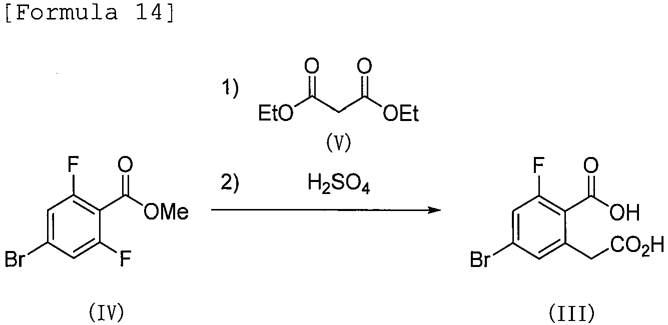

1.5 equivalents of diethyl malonate (V) are added to the compound (IV) in DMSO in the presence of 2 equivalents of cesium carbonate, and reacted at 75 to 85°C for 2 to 4 hours. Then, concentrated sulfuric acid in acetic acid is added and heated at 65 to 75°C for 18 to 24 hours to carry out hydrolysis and decarboxylation reaction to obtain the compound (III). After producing the compound (III), the compound B can be produced according to the description of the above (3).
(5) The compound of the following formula (I) is important as a production intermediate for producing the compound B. The compound can be produced according to the method described in the above (2). 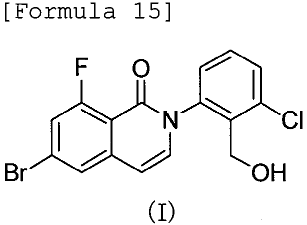
(6) The compound (III) is reacted with acetic anhydride, followed by reaction with the compound (VI) to produce the compound (II), and then the compound (I) is produced according to the production method of the above (2). 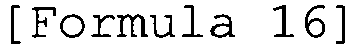 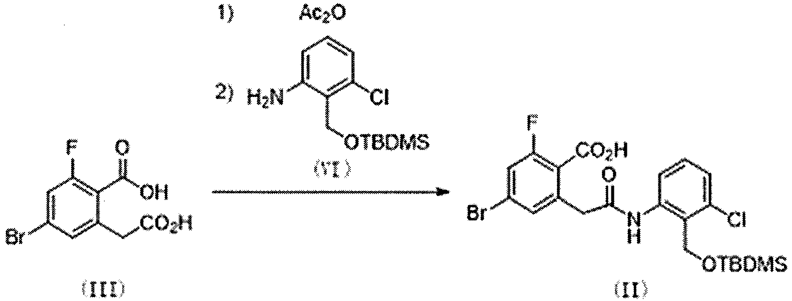 The method for producing the compound (II) from the compound (III) is described in detail in the above (3).
(7) The compound of the formula (IV) is reacted with diethyl malonate (V), and the resulting product is treated with concentrated sulfuric acid to produce the compound (III), and then the compound (I) is produced according to the production method of the above (6). 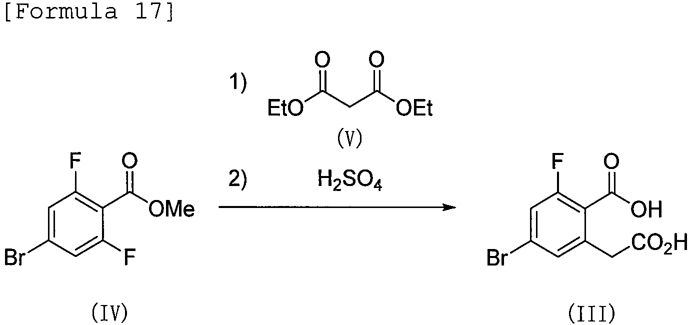 The method for producing the compound (III) from the compound (IV) is described in detail in the above (4).
(8) The compound of the following formula (II) or (III) and a salt thereof are useful as the production intermediates for producing the compound (I). The production methods of these compounds are described in detail in the above (3) and (4).

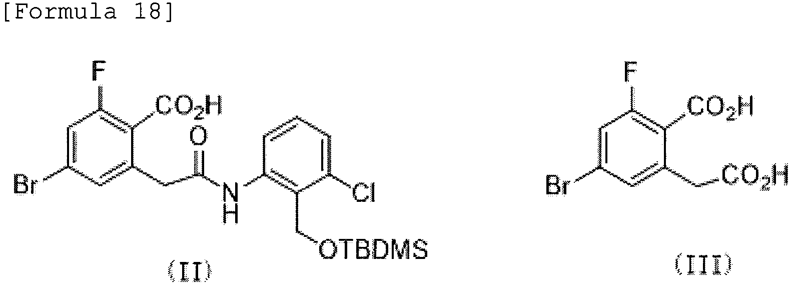

Further, examples of salts of these compounds include alkali metal salts with sodium, potassium, etc., alkaline earth metal salts with magnesium, calcium, etc., organic amine salts with lower alkyl amines, lower alcohol amines, etc., basic amino acid salts with lysine, arginine, ornithine, etc., and ammonium salts, etc. In the case of the compound (III), monocarboxylic and dicarboxylic acid salts are also included.

### Examples

### Example 1

### 2-[3-Chloro-2-(hydroxymethyl)phenyl]-6-cyclopropyl-8-fluoroisoquinolin-1(2H)-one (Compound B)

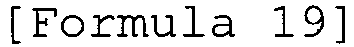

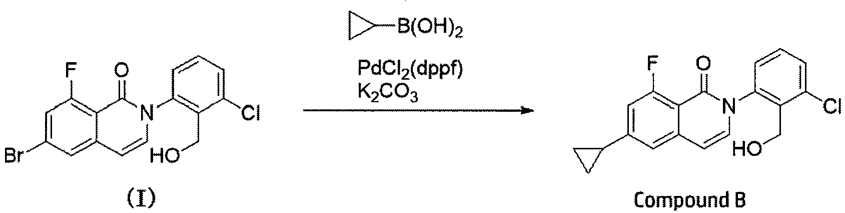

870 g of bromo-derivative (I), 293 g of cyclopropylboronic acid, and 0.97 kg of potassium carbonate were placed in a vessel, and 8.7 L of ethyl acetate and 3.5 L of purified water were added and the temperature was adjusted to 15 to 25°C. The vessel was replaced with nitrogen, 33 g of PdCl₂(dppf) was added, and heat refluxed at 70 to 80°C for 12 hours.

The reaction mixture was cooled to 45 to 55°C, and the insoluble material was filtered and washed with 4.4 L of ethyl acetate. The filtrate and wash liquid were combined, and after confirming dissolution at 60 to 65°C, the aqueous layer was separated. 87 g of activated carbon was added to an organic layer and stirred at 55 to 65°C for 2 hours. The insoluble material was filtered and washed with 4.4 L of ethyl acetate, and the mixed filtrate and the wash liquid were concentrated under reduced pressure until the volume was reduced to about 1/3. Stirring was performed for 12 hours at 15 to 25°C, and after confirmation of crystallization, 8.7 L of n-heptane was added, followed by aging of the crystals at least 2 hours at 15 to 25°C. The resulting product was filtered and washed with 1.74 L of a mixture of heptane-ethyl acetate (2:1) and dried at reduced pressure under a stream of nitrogen. The yield of the compound B was 641 g (82% yield).

¹H NMR (400 MHz, DMSO-d6) δ 7.60 (dd, J = 8.1, 1.2 Hz, 1H), 7.48 (t, J = 8.0 Hz, 1H), 7.32 (dd, J = 7.9, 1.2 Hz, 1H), 7.30 (d, J = 7.4 Hz, 1H), 7.26 (d, J = 1.7 Hz, 1H), 6.99 (dd, J = 13.4, 1.7 Hz, 1H), 6.59 (dd, J = 7.5, 2.1 Hz, 1H), 4.94 (t, J = 5.1 Hz, 1H), 4.50 (dd, J = 11.8, 5.0 Hz, 1H), 4.24 (dd, J = 11.8, 5.4 Hz, 1H), 2.07 (tt, J = 8.4, 5.0 Hz, 1H), 1.16 - 1.01 (m, 2H), 0.91 - 0.78 (m, 2H).
LCMS (m/z): 384.1 [M+H]⁺.

### Example 2

### 6-Bromo-2-[3-chloro-2-(hydroxymethyl)phenyl]-8-fluoroisoquinolin-1(2H)-one (I)

### (1) 4-Bromo-2-(carboxymethyl)-6-fluorobenzoic acid (homophthalic acid) (III)

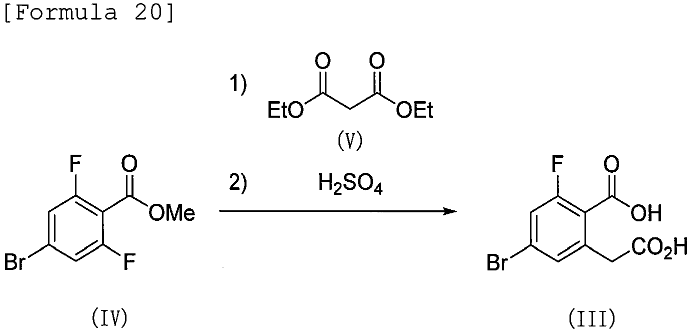

To 7.6 L of DMSO, 3,634 g of cesium carbonate and 1,400 g of methyl 4-bromo-2,6-difluorobenzoate (IV) were added, to which 1,340 g of diethyl malonate (V) was added, and the mixture was heated at 75 to 85°C for 2 hours. At the end of the reaction, 9.8 L of water was added to the reaction mixture and extracted with 12.6 L of toluene. An organic layer was washed with 2.8 L of 15% sodium chloride solution, concentrated under reduced pressure to 8 L or less, and then 19.6 L of acetic acid was added and concentrated again. 3.9 L of concentrated sulfuric acid was added at 35°C or less, and heated at 65 to 75°C for 24 hours. 1.4 L of purified water was added at 35°C or less, and the solid precipitated was filtered off and washed with 4.2 L of a mixture of acetic acid-purified water (2:1), and then washed with purified water until the filtrate reached pH 2.4 or more. Under a stream of nitrogen, drying was performed at 45°C under reduced pressure to give 1,143 g of the target product (III) (74% yield).
¹H NMR (400 MHz, DMSO-d6) δ 13.00 (s, 1H), 8.45 (s, 1H), 7.58 (dd, J = 9.6, 1.9 Hz, 1H), 7.47 (dd, J = 1.9, 0.9 Hz, 1H).
LCMS (m/z): 276.9.

### (2) 4-Bromo-2-{2-([2-{[(tert-butyldimethylsilyl)oxy]methyl}-3-chlorophenyl)amino]-2-oxoethyl}-6-fluorobenzoic acid (II)

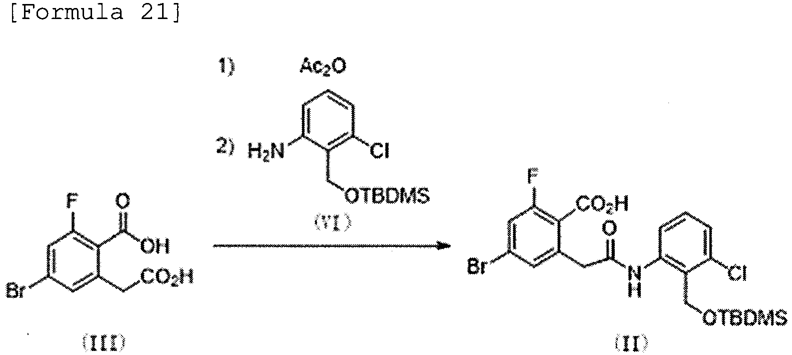

Under a nitrogen atmosphere, 1,140 g of 4-bromo-2-(carboxymethyl)-6-fluorobenzoic acid (III) and 441 g of acetic anhydride were added to 9.1 L of toluene, and heat refluxed for 30 minutes. At the end of the reaction, it was cooled to 15 to 20°C, and 1,175 g of aniline (VI) and 2.28 L of toluene were added to the reaction mixture and stirred at 15 to 25°C for 1 hour. At the end of the reaction, it was cooled to 0 to 5°C, and 5.7 L of n-heptane was added and stirred at the same temperature for 4 hours. The precipitated solid was filtered and washed with 2.3 L of a liquid mixture of heptane-toluene (1:1). Drying was performed under a stream of nitrogen at reduced pressure to give 1,485 g of the target product (II) (68% yield).

¹H NMR (400 MHz, DMSO-d6) δ 13.66 (s, 1H), 9.50 (s, 1H), 8.46 (s, 1H), 7.64 - 7.52 (m, 2H), 7.49 (d, J = 2.1 Hz, 1H), 7.35 - 7.22 (m, 2H), 4.80 (s, 2H), 3.94 (s, 2H), 0.82 (s, 8H), 0.02 (s, 6H).
LCMS (m/z): 416.0 [M+H].

### (3) 6-Bromo-2-[3-chloro-2-(hydroxymethyl)phenyl]-8-fluoroisoquinolin-1(2H)-one (I)

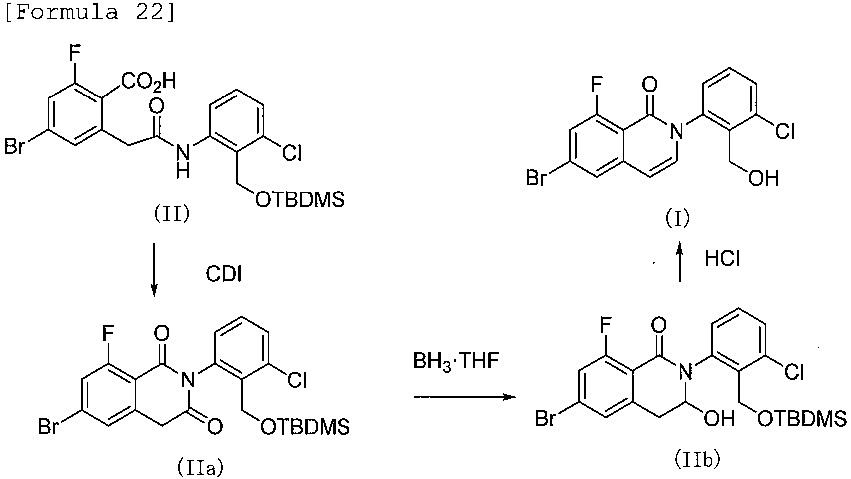

To 1,480 g of the compound (II) in 14.8 L of MeTHF, 1,356 g of CDI was added at 15 to 25°C and stirred for 2 hours. At the end of the reaction, it was washed twice with 14.8 L of 1M hydrochloric acid, followed by washing of the organic layer with 14.8 L of 13% sodium chloride solution until the wash liquid had pH 6 or more. 14.8 L of MeTHF was added to the solution and concentrated to 1/3 volume by reduced pressure distillation. The moisture content of the solution was measured and the process was repeated until the moisture content was 5% or less.

To a separate vessel, 2,396 mL of 1M borane-THF solution was added and cooled to 0 to 5°C, and the above MeTHF solution was added while keeping the temperature at 10°C or less. The reaction mixture was stirred at 0 to 5°C for 3 hours. At the end of the reaction, 3.6 L of methanol was added while keeping the temperature at 0 to 5°C, and the temperature was gradually increased to adjust the mixture to 15 to 25°C. 3.0 L of concentrated hydrochloric acid was added while keeping the temperature of the mixture at 15 to 25°C and stirred for 15 hours. At the end of the reaction, 22.2 L of 13% sodium chloride solution was added while keeping the temperature of the mixture at 15 to 25°C and stirred for 10 minutes, then the mixture was divided and the organic layer was washed with 7.4 L of 13% sodium chloride solution until the wash liquid reached pH 6 or more.

The organic layer was diluted with 14.8 L of toluene and then distilled under reduced pressure, and the process was repeated three times to concentrate the mixture to about 1/2 volume. The temperature of the mixture was adjusted to 40 to 50°C, and 14.8 L of n-heptane was added, then cooled to 0 to 5°C, and then stirred at the same temperature for 2 hours. The precipitated solid was filtered and washed with 5.9 L of a mixture of toluene-heptane (1:1). The resulting product was dried under a stream of nitrogen and reduced pressure to obtain 875 g of the target product (I) (82% yield).

¹H NMR (400 MHz, DMSO-d6) δ 7.90 - 7.85 (m, 1H), 7.67 - 7.55 (m, 2H), 7.49 (t, J = 8.0 Hz, 1H), 7.43 (d, J = 7.4 Hz, 1H), 7.35 (dd, J = 7.9, 1.2 Hz, 1H), 6.69 (dd, J = 7.4, 2.1 Hz, 1H), 4.99 (t, J = 5.1 Hz, 1H), 4.66 - 4.48 (m, 1H), 4.37 - 4.15 (m, 1H).
LCMS (m/z): 382.0 [M+H]⁺.

### Industrial Applicability

According to the present invention, intermediates for producing triazine derivatives having a BTK inhibitory effect can be produced on a semi-industrial or industrial scale, thereby making it possible to establish an industrially advantageous production method of the triazine derivatives and to provide high purity, high quality pharmaceutical products.

## Claims

1. A method for producing a compound B by reacting a compound (I) with a cyclopropylboronic acid in the presence of a palladium catalyst and a base.

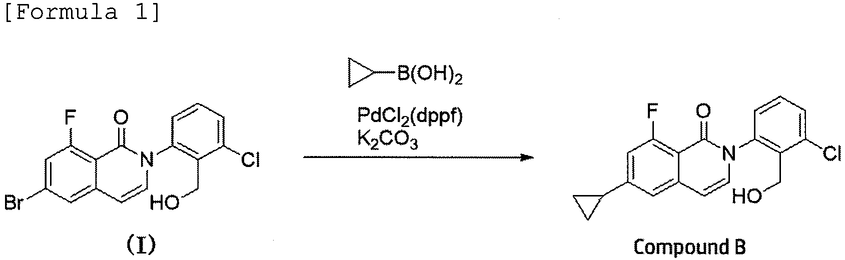

2. The method for producing the compound B according to claim 1, wherein a compound of the formula (II) is reacted with CDI, borane, and concentrated hydrochloric acid in order to produce the compound (I), and then the compound (I) is subjected to the method: 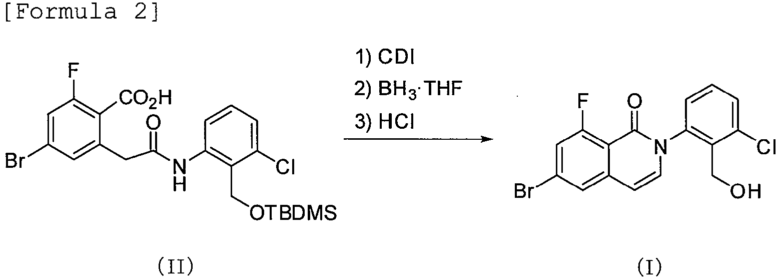 wherein TBDMS stands for tert-butyldimethylsilyl.

3. The method for producing the compound B according to claim 2, wherein a compound (III) is reacted with acetic anhydride, followed by reaction with a compound (VI) to produce the compound (II), and then the compound (II) is subjected to the method: 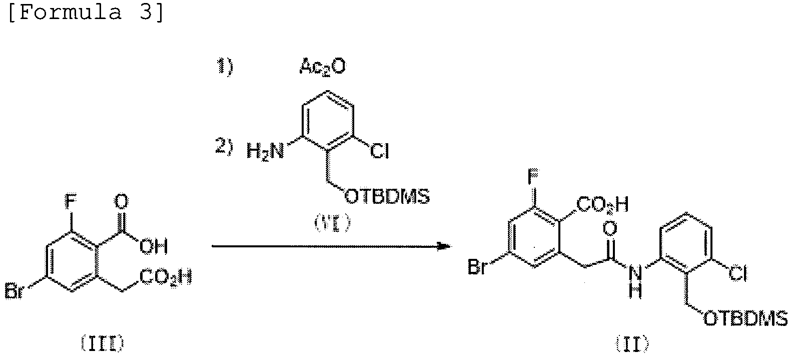 wherein TBDMS is defined as above.

4. The method for producing the compound B according to claim 3, wherein a compound of the formula (IV) is reacted with diethyl malonate (V) and further with concentrated sulfuric acid to produce the compound (III), and then the compound (III) is subjected to the method.

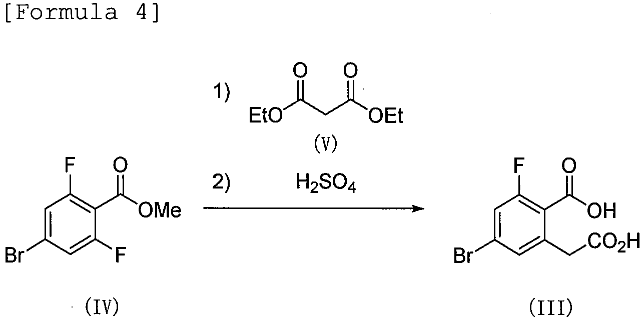

5. A compound represented by the following formula (I).

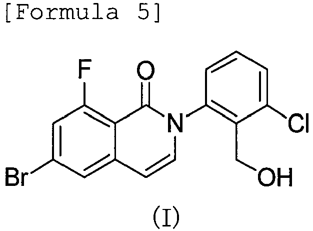

6. A method for producing a compound of the formula (I) by reacting a compound of the formula (II) with CDI, borane, and concentrated hydrochloric acid in order: 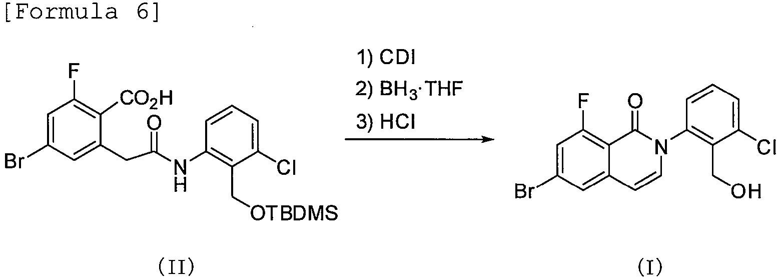 wherein TBDMS is defined as above.

7. The method for producing the compound (I) according to claim 6, wherein a compound (III) is reacted with acetic anhydride, followed by reaction with a compound (VI) to produce the compound (II), and then the compound (II) is subjected to the method: 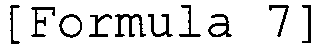 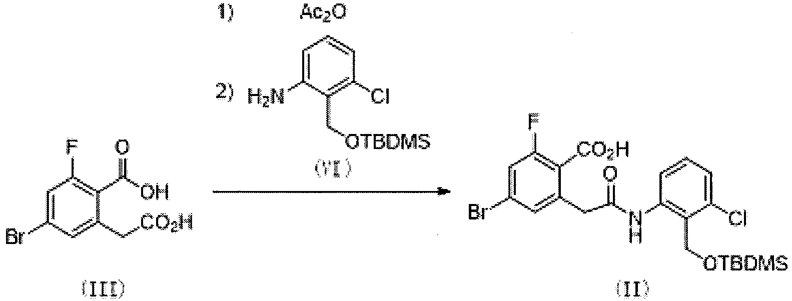 wherein TBDMS is defined as above.

8. The method for producing the compound (I) according to claim 7, wherein a compound (IV) is reacted with diethyl malonate (V), followed by treatment with concentrated sulfuric acid to produce the compound (III), and then the compound (III) is subjected to the method.

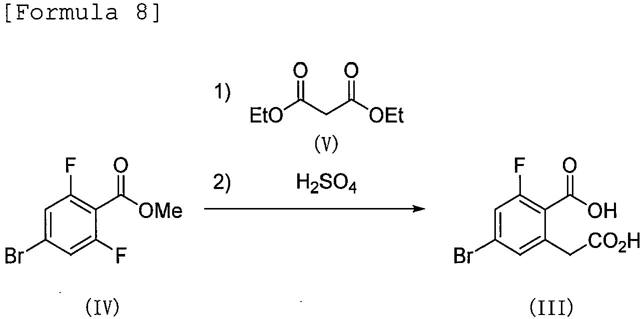

9. A compound of the following formula (II) or (III) and a salt thereof: 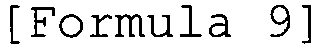 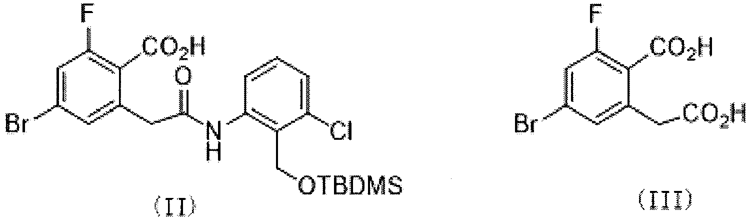 wherein TBDMS is defined as above.
